# FASCICULE DE BREVET EUROPEEN

(11) **EP 1 587 466 B1**
(45) Date de publication et mention de la délivrance du brevet: **18.07.2007**
(21) Numéro de dépôt: 03814509.0
(22) Date de dépôt: 30.12.2003
(51) Int. Cl.: A61F 5/02

(54) **DISPOSITIF POUR LE MAINTIEN DES VERTEBRES LOMBAIRES ET/OU DES MUSCLES SACROSPINAUX**
VORRICHTUNG ZUR UNTERSTÜTZUNG DER LENDENWIRBEL UND/ODER DER VERTEBROSAKRALMUSKEL
DEVICE FOR SUPPORTING LUMBER VERTEBRAS AND/OR VERTEBROSACRAL MUSCLES

(30) Priorité: 30.12.2002 FR 0216835
(43) Date de publication de la demande: 26.10.2005
(73) Titulaire: Parizot, Jean-Paul, 21000 Dijon (FR)
(72) Inventeur: Parizot, Jean-Paul, 21000 Dijon (FR)
(74) Mandataire: Tetaz, Franck Claude Edouard
(86) Numéro de dépôt international: PCT/FR2003/003941
(87) Numéro de publication internationale: WO 2004/060218

(56) Documents cités:
- FR-A- 2 745 490
- US-A- 4 768 499

## Description

La présente invention concerne un dispositif pour le maintien des vertèbres lombaires et/ou des muscles sacrospinaux communément appelés ceinture lombaire.

Dans le domaine des dispositifs orthopédiques, on connaît bien des ceintures dites lombaires destinées à soigner des lombalgies dues à des efforts ponctuels lombopelviens ou à des contraintes répétées sur les structures anatomiques lombopelviennes. Par ailleurs, il est bien connu de prescrire le port de ces ceintures lombaires qui permettent de renforcer la sangle abdominale pour un maintien post-opératoire ou un maintien temporaire de la sangle abdominale lors d'efforts, de périodes de rééducation, etc ...

La plupart de ces ceintures lombaires sont constituées d'une pièce postérieure de maintien lombaire et de deux pièces latérales solidaires de la pièce postérieure et munies à leurs extrémités libres avant de moyens complémentaires de fermeture séparables sur l'abdomen, tels que du Velcro (marque déposée) par exemple. C'est le cas, par exemple, du brevet américain US 4.768.499 qui décrit une ceinture de support des muscles du dos et abdominaux. La ceinture comprend une pièce centrale postérieure non rembourée, obtenue dans du cuir et positionnée dans le creux du dos pour couvrir les cinq vertèbres lombaires et les muscles sacrospinaux de chaque côté des vertèbres lombaires. Les extrémités de la ceinture s'étendent à partir du la pièce centrale postérieure de telle sorte que lorsque lesdites extrémités sont jointes ensemble autour des muscles abdominaux du patient. Ainsi, la pièce centrale postérieure prend appui contre la zone lombaire en pressant vers l'avant dans une position restreignant les muscles sacrospinaux et pressant les vertèbres lombaires afin d'éviter leur relâchement qui pourrait causer une douleur dans la partie inférieure de la colonne vertébrale du patient.

Ces ceintures lombaires présentent l'inconvénient de créer une surépaisseur sur la sangle abdominale lors de leur ouverture, les deux pièces latérales de la ceinture munies de moyens de fermeture du type auto-agrippant, tel que du "Velcro" par exemple, se chevauchant pour assurer la fermeture de ladite ceinture, ce qui crée une gêne pour le patient.

L'un des buts de l'invention est donc de remédier à cet inconvénient en proposant une ceinture lombaire de conception simple et peu onéreuse permettant de fermer la ceinture sans chevauchement des pièces latérales sur la zone abdominale du patient.

A cet effet, et conformément à l'invention, il est proposé un dispositif pour le maintien des vertèbres lombaires et/ou des muscles sacrospinaux, communément appelé ceinture lombaire, et comprenant une pièce postérieure de maintien lombaire et deux pièces latérales solidaires de la pièce postérieure et munies à leurs extrémités libres avants de moyens complémentaires de fermeture remarquable en ce que la face extérieure de la pièce postérieure comprend des moyens de fixation aptes à coopérer avec des moyens de fixation complémentaires solidaires des extrémités libres arrières des pièces latérales afin de fermer la ceinture sans chevauchement des pièces latérales sur la zone abdominale d'un patient.

On comprend bien que, contrairement aux dispositifs de l'art antérieur, le réglage des dimensions de la ceinture lombaire s'effectue sur la pièce postérieure de maintien lombaire, c'est-à-dire dans le dos du patient, et non aux extrémités libres avant des pièces latérales sur la zone abdominale dudit patient.

D'autres avantages et caractéristiques ressortiront mieux de la description qui va suivre d'une variante d'exécution, donnée à titre d'exemple non limitatif du dispositif pour le maintien des vertèbres lombaires conforme à l'invention en référence aux dessins annexés sur lesquels :

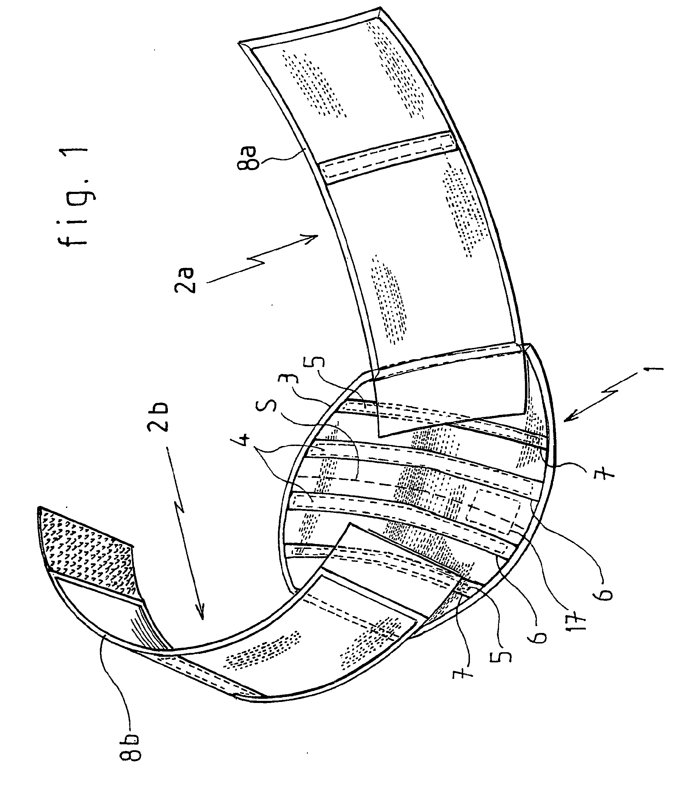

- la figure 1 est une vue en perspective du dispositif pour le maintien des vertèbres lombaires conforme à l'invention,
- la figure 1 est une vue en perspective du dispositif pour le maintien des vertèbres lombaires conforme à l'invention,
- la figure 2 est une vue de dessus des éléments du dispositif de maintien des vertèbres lombaires suivant l'invention mis à plat.

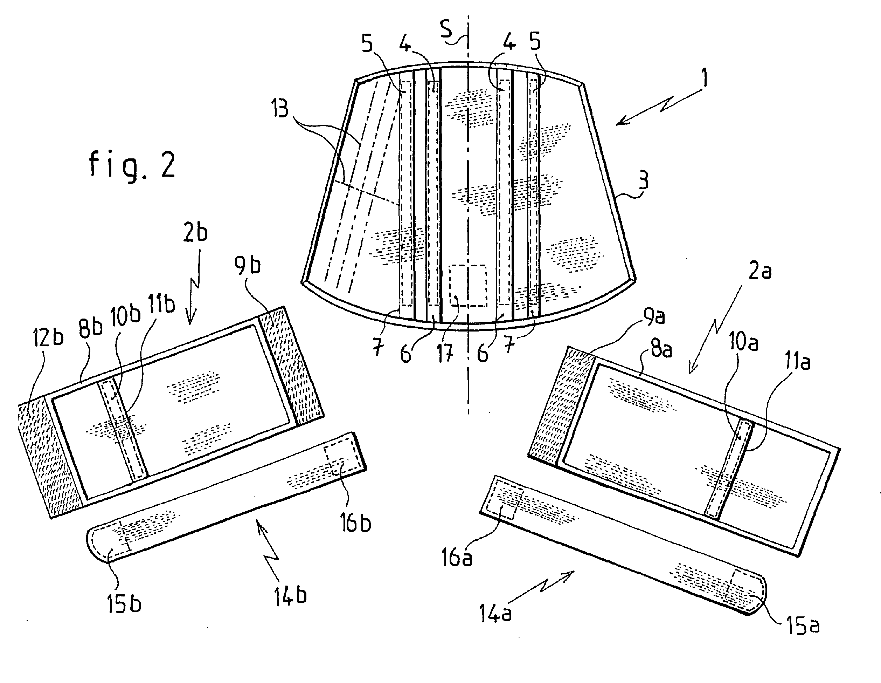

En référence aux figures 1 et 2, le dispositif pour le maintien des vertèbres lombaires ou des muscles sacrospinaux est constitué d'une pièce postérieure de maintien lombaire 1 et de deux pièces latérales 2a et 2b que l'on détaillera plus loin. La pièce postérieure 1 est obtenue dans du tissu, et présente une forme globalement trapézoïdale, la grande et la petite base de la pièce postérieure 1 étant convexe afin d'épouser les courbures du corps du patient sur lequel le dispositif pour le maintien des vertèbres lombaires est mis en place. On notera que la petite et la grande base constituent respectivement la partie supérieure et la partie inférieure de ladite pièce postérieure 1. La pièce postérieure est avantageusement obtenue dans un tissu résilient longitudinalement afin de procurer une contention de la région lombo-pelvienne. Par ailleurs, ladite pièce postérieure 1 comprend à sa périphérie une ganse 3 afin d'empêcher son effilochage, ladite ganse 3 étant également légèrement résiliente. La pièce postérieure 1 présente sur sa face extérieure, c'est-à-dire la face de la pièce postérieure 1 qui ne prend pas appui sur le corps du patient, des boucles aptes à coopérer avec des crochets complémentaires de moyens de fermeture du type Velcro (marque déposée) solidaires de l'extrémité libre arrière des pièces latérales 2a et 2b.

Par ailleurs, la pièce postérieure 1 comprend, par ailleurs, quatre baleines transversales s'étendant parallèlement les unes par rapport aux autres de la petite à la grande base de la pièce postérieure 1 et uniformément répartie de part et d'autre de l'axe de symétrie S de ladite pièce postérieure 1. Ainsi, la pièce postérieur 1 comprend deux baleines centrales 4 et deux baleines extérieures 5 s'étendant dans des fourreaux respectivement 6 et 7 cousus sur la face extérieure de la pièce postérieure 1 afin de ne pas occasionner de gêne pour le patient. On observera que l'on entend par baleines centrales 4 les baleines s'étendant à proximité de l'axe de symétrie S de la pièce postérieure 1. Les fourreaux 7 des baleines extérieures 5 sont obtenus dans un tissu présentant des boucles aptes à coopérer avec des moyens de fermeture à crochets des pièces latérales 2a, 2b comme on le verra plus loin. Par ailleurs, les fourreaux 6 des baleines centrales 4 sont obtenus dans une matière lisse de manière à ce que les pièces latérales 2a,2b ne puissent pas être solidarisées au droit desdites baleines centrales 4. Par ailleurs, les baleines centrales 4 et extérieures 5 de la pièce postérieure 1 sont avantageusement courbées de telle sorte que la face extérieure de la pièce postérieure 1 soit concave et que la face intérieure, c'est-à-dire la face prenant appui sur les lombaires du patient, de ladite pièce postérieure 1 soient convexes afin d'épouser la cambrure naturelle de la partie inférieure du dos.

Les pièces latérales 2a,2b, en référence aux figures 1 et 2, sont respectivement constituées d'une bande de tissu rectangulaire munie à sa périphérie d'une ganse 8a et respectivement 8b afin d'empêcher son effilochage. Chaque pièce latérale 2a, 2b comprend à l'une de ses extrémités dite extrémité libre arrière une bande de tissu 9a et respectivement 9b, munie de crochets aptes à coopérer avec les boucles de la face extérieure de la pièce postérieure 1. Ces bandes de tissu 9a, 9b sont cousues aux extrémités libres arrières des pièces latérales 2a et respectivement 2b. Chacune des pièces latérales 2a, 2b comprend à proximité de son extrémité libre avant une baleine transversale dite de maintien abdominal 10a et respectivement 10b qui s'étend dans un fourreau respectivement 11a et 11b cousu sur la lace extérieure de la pièce latérale respectivement 2a et 2b. De la même manière que précédemment, les pièces latérales 2a et 2b sont avantageusement obtenues dans du tissu présentant sur sa face extérieure, c'est-à-dire la face des pièces latérales 2a et 2b qui ne rentre pas en contact avec la peau du patient, des boucles aptes à coopérer avec des moyens complémentaires de fermeture a crochets du type Velcro (marque déposée). Par ailleurs, ce tissu est avantageusement résilient longitudinalement.

L'une des pièces latérales 2a,2b, dans cet exemple particulier de réalisation (en l'espèce la pièce latérale gauche 2b), comprend à son extrémité libre avant une bande de tissu 12b munie de moyens complémentaires de fermeture du type à crochets cousue à ladite extrémité libre de la pièce latérale 2b. Ainsi, les pièces latérales 2a, 2b étant solidarisées à la pièce postérieure 1 par les moyens de fermeture 9a, 9b de telle sorte que les bords transversaux des pièces 2a,2b s'étendent parallèlement aux bords transversaux de la pièce postérieure 1, les extrémités libres des pièces latérales 2a et respectivement 2b étant de préférence positionnées entre les baleines centrales 4 et les bords transversaux de ladite pièce postérieure 1. On observera que les extrémités arrières des pièces latérales ne peuvent être solidarisées à la pièce postérieure 1 au-delà des baleines centrales 4 dont le fourreau 6 obtenu dans une matière lisse empêche la solidarisation des pièces latérales 2a et respectivement 2b.

De manière particulièrement avantageuse, la face extérieure de la pièce postérieure 1 comprend des lignes de repairage 13 s'étendant parallèlement et/ou perpendiculairement aux bords latéraux de la pièce postérieure 1, lesdites lignes étant représentées en traits mixtes sur la figure 2. Ces lignes permettent au patient de s'assurer du bon alignement ou du bon parallélisme des bords transversaux des pièces latérales 2a et 2b par rapport aux bords transversaux de la pièce postérieure 1.

Accessoirement, le dispositif pour le maintien des vertèbres lombaires et/ou des muscles sacrospinaux comprend deux pièces latérales secondaires 14a, 14b constituées de deux bandes de tissu étroites rectangulaires obtenues dans un tissu élastique longitudinalement et muni sur l'une de ses faces, correspondant à la face extérieure de ces pièces latérales secondaires 14a,14b, de boucles aptes à coopérer avec des moyens de fermeture complémentaires à crochets. Ces pièces latérales secondaires 14a, 14b, présentent une longueur légèrement inférieure à la longueur des pièces latérales 2a et 2b. De plus, ces pièces latérales secondaires 14a,14b comprennent sur leur face intérieure, à leurs extrémités libres avant légèrement arrondie, et arrière, des moyens de fixation autoagrippant 15a, 16a et respectivement 15b, 16b du type à crochets aptes à coopérer avec les boucles de la face extérieure desdites pièces latérales secondaires 14a,14b et/ou des pièces latérales 2a, 2b et/ou des boucles de la face extérieure et la pièce postérieure. Ainsi, une première pièce latérale secondaire est solidarisée dans la partie inférieure de la pièce postérieure 1, entre les baleines centrales 4, dans une zone 17 représentée en traits pointillés sur la figure 2 de telle sorte que la première pièce latérale 14a s'étende globalement perpendiculairement au bord transversal droit de la pièce postérieure 1, c'est-à-dire parallèlement à la pièce latérale 2a. L'extrémité arrière de la seconde pièce latérale secondaire 14b est alors solidarisée sur la face supérieure de la première pièce latérale secondaire 14a au-dessus de la zone 17 d'accrochage de la pièce postérieure 1 de telle sorte que la seconde pièce latérale secondaire 14b s'étende perpendiculaire au second bord latéral gauche de la pièce postérieure 1 parallèlement à la pièce latérale 2b et, les pièces latérales 2a,2b et les pièces latérales 14a,14b s'étendent symétriquement par rapport à l'axe de symétrie S de ladite pièce postérieure 1. Les extrémités libres avant des pièces latérales secondaires 14a,14b sont alors solidarisées sur la face extérieure des pièces latérales 2a, 2b de telle sorte que lesdites pièces latérales secondaires 14a, 14b soient tendues afin de procurer un point de pression supplémentaire en regard de la zone 17 sur les vertèbres lombaires du patient.

Il va de soi que les moyens de fixation et les moyens de fixation complémentaires de la pièce postérieure 1, des pièces latérales 2a, 2b et des pièces latérales secondaires 14a, 14b peuvent consister dans tout moyen de fixation connu tel que des moyens de fixation auto-agrippant, crochet/crochet ou analogue.

Par ailleurs, il est bien évident que les boucles des faces extérieures de la pièce postérieur 1 et/ou des pièces latérales principales 2a, 2b et/ou secondaires 14a,14b peuvent consister dans des boucles d'une bande de tissu cousue sur la face extérieure desdites pièces sans sortir du cadre de l'invention.

Enfin, il va de soi que les exemples que l'on vient de donner ne sont que des illustrations particulières et que l'on pourrait concevoir diverses variantes du dispositif pour le maintien des vertèbres lombaires sans pour autant sortir du cadre de l'invention.

## Revendications

1. - Dispositif pour le maintien des vertèbres lombaires et/ou des muscles sacrospinaux, communément appelés ceinture lombaire, et comprenant une pièce postérieure de maintien lombaire (1) et deux pièces latérales (2a, 2b) solidaires de la pièce postérieure (1) et munies à leurs extrémités libres avants de moyens complémentaires (12b) de fermeture **caractérisé en ce que** la face extérieure de la pièce postérieure (1) comprend des moyens de fixation aptes à coopérer avec des moyens de fixation complémentaires (9a,9b) solidaires des extrémités libres arrières des pièces latérales (2a,2b) afin de fermer la ceinture sans chevauchement des pièces latérales (2a,2b) sur la zone abdominale d'un patient.

2. - Dispositif suivant la revendication précédente **caractérisé en ce que** la pièce postérieure (1) présente une forme globalement trapézoïdale, la grande et la petite base dudit trapèze étant convexes, munies d'au moins quatre baleines, deux baleines centrales (4) et deux baleines extérieures (5) s'étendant transversalement de la petite à la grande base et réparties de part et d'autre de l'axe de symétrie (S) de la pièce postérieure (1).

3. - Dispositif suivant la revendication 2 **caractérisé en ce que** les baleines centrales (4) sont solidarisées sur la face extérieure de la pièces postérieure (1) par un fourreau (6) obtenu dans une matière lisse de manière que les pièces latérales (2a, 2b) ne puissent pas être solidarisées sur lesdites baleines centrales (4).

4. - Dispositif suivant l'une quelconque des revendications précédentes **caractérisé en ce que** chaque pièce latérale (2a,2b) comprend à proximité de son extrémité avant au moins une baleine transversale (10a,10b) de maintien abdominal.

5. - Dispositif suivant l'une quelconque des revendications précédentes **caractérisé en ce qu'**il comprend deux pièces latérales secondaires (14a,14b) comprenant à ses extrémités libres, sur sa face intérieure (15a,16a;15b,16b), des moyens de fixation aptes à coopérer d'une part avec des moyens de fixation complémentaires positionnés sur la face extérieure desdites pièces latérales secondaires (14a,14b) et/ou des pièces latérales principales (2a,2b) et d'autre part avec les moyens de fixation complémentaire de la face extérieure de la pièce postérieure (1).

6. - Dispositif suivant l'une quelconque des revendications précédentes **caractérisé en ce que** la pièce postérieure et/ou les pièces latérales principales (2a,2b) et/ou secondaires (14a,14b) sont obtenues dans un tissu élastique longitudinalement.

7. - Dispositif suivant l'une quelconque des revendications 2 à 6 **caractérisé en ce que** les baleines centrales (4) et extérieures (5) de la pièce postérieure (1) sont courbées de sorte que la face extérieure de la pièce postérieure (1) soit concave et que la face intérieure de ladite pièce postérieure (1) qui prend appui sur les vertèbres lombaires du patient soit convexe.

8. - Dispositif suivant l'une quelconque des revendications précédentes **caractérisé en ce que** les moyens de fixation de la face extérieure de la pièce postérieure (1) et/ou des pièces latérales principales (2a,2b) et/ou secondaires (14a,14b) et les moyens de fixation complémentaires (9a,9b;15a,16a;15b,16b;12b) consistent dans des moyens de fixation du type boucle/crochet ou crochet/crochet.

## Claims

1. Device for supporting lumbar vertebras and / or sacrospinal muscles commonly called a lumbar belt, and composed of a posterior lumbar support part (1) and two lateral parts (2a, 2b) fixed to the posterior part (1) and provided with additional closing means (12b) at their front free ends, **characterised in that** the outside face of the posterior part (1) comprises fixing means capable of cooperating with additional fixing means (9a, 9b) connected to the free back ends of the lateral parts (2a, 2b) in such a way that it is possible to close the belt without overlapping the lateral parts (2a, 2b) on the abdominal region of a patient.

2. Device according to the previous claim, **characterised in that** the posterior part (1) has a globally trapezoidal shape, the large and the small base of the said trapezoid being convex, provided with at least four whalebones, two central whalebones (4) and two external whalebones (5) extending transversally from the small base to the large base and distributed on each side of the axis of symmetry (S) of the posterior part (1).

3. Device according to claim 2, **characterised in that** the central whalebones (4) are fixed on the outside face of the posterior part (1) by a sheath (6) obtained from a smooth material so as to prevent fixing of the lateral parts (2a, 2b) on the said central whalebones (4).

4. Device according to any one of the previous claims, **characterised in that** each lateral part (2a, 2b) comprises at least one transverse whalebone (10a, 10b) close to its front end for abdominal support.

5. Device according to any one of the previous claims, **characterised in that** it comprises two secondary lateral parts (14a, 14b) comprising attachment means on its free ends, on its inside face (15a, 16a, 15b, 16b) that can cooperate firstly with additional attachment means on the outside face of the said secondary lateral parts (14a, 14b) and / or principal lateral parts (2a, 2b), and secondly with the additional attachment means of the outside face of the posterior part (1).

6. Device according to any one of the previous claims, **characterised in that** the posterior part and / or the principal lateral parts (2a, 2b) and / or the secondary lateral parts (14a, 14b) are obtained from a longitudinally elastic fabric.

7. Device according to any one of claims 2 to 6, **characterised in that** the central (4) and external (5) whalebones of the posterior part (1) are curved such that the outside face of the posterior part (1) is concave and the inside face of the said posterior part (1) that bears on the patient's lumbar vertebras is convex.

8. Device according to any one of the previous claims, **characterised in that** the attachment means of the outside face of the posterior part (1) and / or the principal lateral parts (2a, 2b) and / or the secondary lateral parts (14a, 14b) and the additional attachment means (9a, 9b, 15a, 16a, 15b, 16b, 12b) consist of attachment means of the loop / hook or hook / hook type.

## Patentansprüche

1. Vorrichtung zur Stützung von Lumbalwirbeln und/oder von sacrospinalen Muskeln, allgemein Lumbalgürtel genannt, die ein hinteres Teil (1) zur Lumbalstützung und zwei seitliche Teile (2a, 2b) umfasst, die fest mit dem hinteren Teil (1) verbunden sind und an ihren vorderen, freien Enden mit komplementären Schließmitteln (12b) versehen sind, **dadurch gekennzeichnet, dass** die äußere Fläche des hinteren Teils (1) Befestigungsmittel umfasst, die geeignet sind, mit komplementären Befestigungsmitteln (9a, 9b) zusammenzuwirken, die fest mit den hinteren, freien Enden der seitlichen Teile (2a, 2b) verbunden sind, um den Gürtel ohne Überdeckung der seitlichen Teile (2a, 2b) auf einer Bauchzone eines Patienten zu schließen.

2. Vorrichtung nach dem vorhergehenden Anspruch, **dadurch gekennzeichnet, dass** das hintere Teil (1) eine insgesamt trapezförmige Gestalt aufweist, wobei die große und die kleine Basis des genannten Trapezes konvex sind und mit mindestens vier Versteifungen versehen ist, zwei mittleren Versteifungen (4) und zwei äußeren Versteifungen (5), die sich quer von der kleinen zur großen Basis erstrecken und beiderseits der Symmetrieachse (S) des hinteren Teils (1) verteilt sind.

3. Vorrichtung nach Anspruch 2, **dadurch gekennzeichnet, dass** die mittleren Versteifungen (4) auf der Außenfläche der hinteren Teile (1) durch eine Hülse (6) fest angebracht sind, die aus einem glatten Material erhalten wurde, so dass die seitlichen Teile (2a, 2b) nicht auf den genannten mittleren Versteifungen (4) fest angebracht werden können.

4. Vorrichtung nach irgendeinem der vorhergehenden Ansprüche, **dadurch gekennzeichnet, dass** jedes seitliche Teil (2a, 2b) in der Nähe seines vorderen Endes mindestens eine querverlaufende Versteifung (10a, 10b) zur abdominalen Stützung umfasst.

5. Vorrichtung nach irgendeinem der vorhergehenden Ansprüche, **dadurch gekennzeichnet, dass** sie zwei seitliche Sekundärteile (14a, 14b) umfasst, die an ihren freien Enden auf der inneren Fläche (15a, 16a; 15b, 16b) Befestigungsmittel umfassen, die geeignet sind, einerseits mit komplementären Befestigungsmitteln, die auf der äußeren Fläche der genannten seitlichen Sekundärteile (14a, 14b) und/oder der seitlichen Hauptteile (2a, 2b) positioniert sind, und andererseits mit den komplementären Befestigungsmitteln der äußeren Fläche des hinteren Teils (1) zusammenzuwirken.

6. Vorrichtung nach irgendeinem der vorhergehenden Ansprüche, **dadurch gekennzeichnet, dass** das hintere Teil und/oder die seitlichen Hauptteile (2a, 2b) und/oder Sekundärteile (14a, 14b) aus einem in Längsrichtung elastischen Textilerzeugnis erhalten sind.

7. Vorrichtung nach irgendeinem der Ansprüche 2 bis 6, **dadurch gekennzeichnet, dass** die mittleren (4) und äußeren (5) Versteifungen des hinteren Teils (1) derart gekrümmt sind, dass die äußere Fläche des hinteren Teils (1) konkav ist und dass die innere Fläche des genannten hinteren Teils (1), die auf den Lumbalwirbeln des Patienten aufliegt, konvex ist.

8. Vorrichtung nach irgendeinem der vorhergehenden Ansprüche, **dadurch gekennzeichnet, dass** die Befestigungsmittel der äußeren Fläche des hinteren Teils (1) und/oder der seitlichen Hauptteile (2a, 2b) und/oder Sekundärteile (14a, 14b) und die komplementären Befestigungsmittel (9a, 9b; 15a, 16a; 15b, 16b; 12b) aus Befestigungsmitteln der Art Schlinge/Haken oder Haken/Haken bestehen.
